(19) Europäisches Patentamt
European Patent Office
Office européen des brevets

(11) Veröffentlichungsnummer: **0 269 914 B1**

(12) **EUROPÄISCHE PATENTSCHRIFT**

(45) Veröffentlichungstag der Patentschrift: **12.06.91**

(51) Int. Cl.$^5$: **A61K 7/13**, C07C 215/76, C07C 217/84, C07C 213/02

(21) Anmeldenummer: **87116491.9**

(22) Anmeldetag: **09.11.87**

(54) **Mittel und Verfahren zur oxidativen Färbung von Haaren mit 3-(2',2',2'-Trifluorethyl)amino-phenolderivaten sowie neue 3-(2',2',2'-Trifluorethyl)amino-phenolderivate.**

(30) Priorität: **04.12.86 DE 3641630**

(43) Veröffentlichungstag der Anmeldung:
**08.06.88 Patentblatt 88/23**

(45) Bekanntmachung des Hinweises auf die Patenterteilung:
**12.06.91 Patentblatt 91/24**

(84) Benannte Vertragsstaaten:
**AT BE CH DE ES FR GB GR IT LI NL SE**

(56) Entgegenhaltungen:
**EP-A- 0 008 080**
**LU-A- 56 491**

**CHEMICAL ABSTRACTS, Band 90, Nr. 1, 1. Januar 1979, Seite 554, Zusammenfassung Nr. 6058e, Columbus, Ohio, US; E.R. BISSELL et al.: "Synthesis of some trifluoroethyl-substituted derivatives of aniline", & J. FLUORINE CHEM. 1978, 12(4), 293-305**

(73) Patentinhaber: **Wella Aktiengesellschaft**
**Berliner Allee 65**
**W-6100 Darmstadt(DE)**

(72) Erfinder: **Braun, Hans-Jürgen, Dr.**
**Im Dorf 32**
**CH-3183 Albligen(CH)**

EP 0 269 914 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein Mittel und ein Verfahren zur oxidativen Färbung von Haaren auf der Basis einer Entwicklersubstanz-Kupplersubstanz-Kombination, worin als Kupplersubstanz ein 3-(2′,2′,2′-Trifluorethyl)amino-phenolderivat verwendet wird, sowie neue 3-(2′,2′,2′-Trifluorethyl)amino-phenolderivate.

In der Haarfärbepraxis haben die Oxidationsfarbstoffe eine wesentliche Bedeutung erlangt. Hierbei werden die Farbstoffe durch oxidative Kupplung von Entwicklersubstanzen und Kupplersubstanzen im Haarschaft erzeugt. Dies führt zu sehr intensiven Haarfärbungen mit sehr guter Farbechtheit. Außerdem können durch die Kombination geeigneter Entwickler- und Kupplersubstanzen unterschiedliche Farbnuancen erzeugt werden.

Als Entwicklersubstanzen werden üblicherweise 1,4-Diamino-benzol, 2,5-Diamino-toluol, 2,5-Diamino-anisol sowie 4-Amino phenol verwendet. Eine gewisse Bedeutung haben auch 2,5-Diamino-benzylalkohol und 2-(2′,5′-Diamino)phenyl-ethanol erlangt.

Diese Entwicklersubstanzen ergeben mit geeigneten Kupplersubstanzen unter Einwirkung eines Oxidationsmittels die Haarfärbungen. So erhält man mit den Kupplersubstanzen Resorcin, 2-Methyl-resorcin oder 4-Chlor-resorcin naturblond bis hellbraun gefärbte Haare.

Durch die Kombination der Entwicklersubstanzen 1,4-Diamino-benzol beziehungsweise 4-Amino-phenol mit den Kupplersubstanzen 5-Amino-2-methyl-phenol beziehungsweise 1,3-Diamino-benzolderivaten wie 2,4-Diamino-anisol sind auch rote Haarfärbungen erhältlich.

Blaue bis blauviolette Farbtöne werden mit 1,4-Diamino-benzol oder dessen Derivaten, wie zum Beispiel 2,5-Diamino-toluol, als Entwicklersubstanz hergestellt, wobei man auf die Verwendung von 1,3-Diamino-benzolderivaten als Kupplersubstanz angewiesen ist.

Schwarze Haarfärbungen können durch die Kombination der vorstehend genannten Entwickler- und Kupplersubstanzen mit weiteren geeigneten Kupplersubstanzen erhalten werden.

Die 1,3-Diamino-benzolderivate werden jedoch als toxikologisch bedenklich beurteilt, so daß ein Bedarf für einen Ersatz dieser Stoffe in Haarfärbemitteln besteht.

Es ist schon mehrfach versucht worden, die 1,3-Diamino-benzolderivate durch die im allgemeinen toxikologisch besser beurteilten 3-Amino-phenolderivate zu ersetzen.

Mit den bekannten Kupplersubstanzen 3-Amino-phenol und 5-Amino-2-methyl-phenol ist es jedoch nur möglich, rote Haarfärbungen zu erzeugen. Ferner erhält man mit der in der DE-OS 2 429 780 beschriebenen Kupplersubstanz 5-Amino-2-chlor-phenol in Kombination mit der Entwicklersubstanz 2,5-Diamino-toluol ein rotstichiges Violett. Das 5-Amino-2-chlor-phenol ist daher als Ersatz für 1,3-Diamino-benzol nicht vollwertig geeignet.

Aufgabe der Erfindung ist es daher, ein Haarfärbemittel auf der Basis einer Entwicklersubstanz-Kupplersubstanz-Kombination zur Verfügung zu stellen, worin die Kupplersubstanz toxikologisch günstiger als 1,3-Diamino-benzol ist und bessere färberische Eigenschaften aufweist als die bekannten 3-Amino-phenolderivate.

Hierzu wurde nun gefunden, daß ein Mittel zur oxidativen Färbung von Haaren auf der Basis einer Entwicklersubstanz-Kupplersubstanz-Kombination, welches als Kupplersubstanz ein 3-(2′,2′,2′-Trifluorethyl)-amino-phenolderivat der allgemeinen Formel (I)

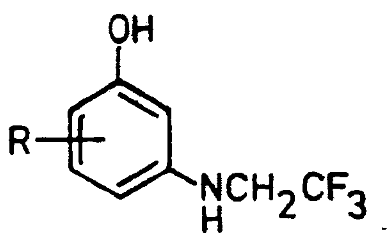

(I),

worin der Rest R

- einen Alkylrest mit 1 bis 4 Kohlenstoffatomen,
- einen Mono- oder Polyhydroxyalkylrest mit 1 bis 4 Kohlenstoffatomen und bis zu 3 Hydroxylgruppen,
- einen Alkoxyrest mit 1 bis 4 Kohlenstoffatomen,
- einen Mono- oder Polyhydroxyalkoxyrest mit 1 bis 4 Kohlenstoffatomen und bis zu 3 Hydroxylgruppen oder
- einen Halogenrest

darstellt, oder dessen physiologisch verträgliches Salz enthält, die gestellte Aufgabe in hervorragender Weise löst.

Der Rest R in der allgemeinen Formel (I) kann hierbei in einer ortho-, meta- oder para-Stellung zum (2',2',2'-Trifluorethyl)aminorest stehen, wobei die para-Stellung zum (2',2',2'-Trifluorethyl)aminorest bevorzugt ist. Als Halogenrest ist Chlor bevorzugt.

Als physiologisch verträgliches Salz mit anorganischen oder organischen Säuren seien beispielsweise das Chlorid, das Sulfat, das Phosphat, das Acetat, das Proprionat, das Lactat und das Citrat genannt.

Gegenüber den bekannten 3-Amino-phenolderivaten wurde überraschend gefunden, daß beispielsweise mit der neuen Kupplersubstanz 2-Chlor-5-(2',2',2'-trifluorethyl)amino-phenol in Kombination mit den Entwicklersubstanzen 1,4-Diamino-benzol, 2,5-Diamino-toluol, 2,5-Diamino-benzylalkohol oder 2-(2',5'-Diamino)-phenyl-ethanol reine blaue Färbungen der Haare ohne Rotanteil erhalten werden. Die Verbindung 2-Chlor-5-(2',2',2'-trifluorethyl)amino-phenol stellt somit das erste 3-Amino-phenolderivat mit den Färbeeigenschaften einer typischen Blaukupplersubstanz dar. In Kombination mit der Entwicklersubstanz 4-Amino-phenol färbt 2-Chlor-5-(2 ,2',2'-trifluorethyl)amino-phenol die Haare in einem reinen Rot.

Diese Färbemöglichkeit besteht mit der bekannten Kupplersubstanz 5-Amino-2-chlor-phenol nicht Die Kombination von 5-Amino-2-chlor-phenol mit 4-Aminophenol als Entwicklersubstanz ergibt nur einen rotbraunen Farbton.

Die neue Kupplersubstanz 2-Chlor-5-(2',2',2'-trifluoretyhl)amino-phenol besitzt somit die vorteilhaften färberischen Eigenschaften eines 1,3-Diamino-benzolderivates und weist darüberhinaus die gegenüber den 1,3-Diamino-benzolderivaten besseren toxikologischen Eigenschaften der 3-Amino phenolderivate auf. Es ist daher möglich, die bisher in Haarfärbemitteln verwendeten toxikologisch bedenklichen 1,3-Diamino-benzolderivate vorteilhaft zu ersetzen.

Die ebenfalls neuen Kupplersubstanzen der Formel (I) 2-Methyl-5-(2',2',2'-trifluorethyl)amino phenol, 2-Methyl-3-(2',2',2'-trifluorethyl)amino-phenol und 2-Methoxy-5-(2',2',2'-trifluorethyl)amino-phenol färben die Haare in Kombination mit den Entwicklersubstanzen 1,4-Diamino-benzol, 2,5-Diamino-toluol, 2,5-Diamino-benzylalkohol oder 2-(2',5'-Diamino)phenyl-ethanol in weinroten bis rotvioletten Farbtönen ein und stellen eine Erweiterung der Färbemöglichkeiten im Bereich von modischen Rottönen dar.

In dem erfindungsgemäßen Haarfärbemittel soll die Kupplersubstanz der Formel (I) in einer Menge von etwa 0,01 bis 4,0 Gewichtsprozent, vorzugsweise 0,02 bis 2,0 Gewichtsprozent, enthalten sein.

Die Kupplersubstanz der Formel (I) wird im allgemeinen in etwa äquimolarer Menge, bezogen auf die verwendeten Entwicklersubstanzen, eingesetzt Wenn sich auch der äquimolare Einsatz als zweckmäßig erweist, so ist es doch nicht nachteilig, wenn die Kupplersubstanz in einem gewissen überschuß oder Unterschuß zum Einsatz kommt. Es ist ferner nicht notwendig, daß die Entwicklerkomponente und die Kupplerkomponente einheitliche Produkte darstellen, vielmehr kann sowohl die Entwicklerkomponente ein Gemisch von bekannten Entwicklersubstanzen als auch die Kupplerkomponente ein Gemisch der erfindungsgemäßen Verbindung mit bekannten Kupplersubstanzen darstellen.

Von den bekannten Entwicklersubstanzen kommen als Bestandteil der erfindungsgemäßen Haarfärbemittel vor allem 1,4-Diamino-benzol, 2,5-Diamino-toluol, 2,5-Diamino-anisol, 4-Amino-phenol, 2,5-Diamino-benzylalkohol und 2-(2',5'-Diamino)phenyl-ethanol in Betracht. Es können selbstverständlich auch noch andere Entwicklersubstanzen, wie zum Beispiel 3-Methyl-4-amino-phenol oder Tetraaminopyrimidin, verwendet werden.

Außerdem können in dem Haarfärbemittel zusätzlich bekannte Kupplersubstanzen, insbesondere Resorcin, 4-Chlor-resorcin, 4,6-Dichlor-resorcin, 2-Methyl-resorcin, 2-Amino-4-(2'-hydroxyethyl)amino-anisol, 1,5-Dihydroxy-tetralin, 3-Amino-2-methyl-phenol, 2,4-Diamino-anisol, 2,4-Diamino-benzylalkohol, 5-Amino-2-methyl-phenol, 2,4-Diamino-phenetol, 1,3 Diamino-benzol, 1-Naphthol, 4-Hydroxy-indol, 5-Hydroxy-benzodioxol-(1,3), 5-Amino-benzodioxol-(1,3), 5-(2'-Hydroxyethyl)amino-benzodioxol-(1,3), 2,4-Dihydroxy-anisol und 2,4-Dihydroxy-phenoxyethanol, allein oder im Gemisch miteinander, enthalten sein.

Derartige zur Haarfärbung bekannte und übliche Oxidationsfarbstoffe sind unter anderem in dem Buch von E. Sagarin, "Cosmetics, Science and Technology", Interscience Publishers Inc., New York (1957), Seiten 503 ff. sowie in dem Buch von H. Janistyn, "Handbuch der Kosmetika und Riechstoffe" (1973), Seiten 338 ff. beschrieben.

Die Gesamtmenge der in dem beanspruchten Haarfärbemittel enthaltenen Entwicklersubstanz-Kupplersubstanz-Kombination soll etwa 0,1 bis 6,0 Gewichtsprozent, vorzugsweise 0,5 bis 3,0 Gewichtsprozent betragen.

Zur Erzielung gewisser Farbnuancen können ferner auch übliche direktziehende Farbstoffe, beispielsweise Triphenylmethanfarbstoffe wie Diamond Fuchsine (C.I. 42 510) und Leather Ruby HF (C.I. 42 520), aromatische Nitrofarbstoffe wie zum Beispiel 2-(2'-Hydroxyethyl)amino-4,6-dinitro-phenol, 4-(2'-Hydroxyethyl)amino-2-nitro-anilin und 2-Amino-4-nitro-phenol, Azofarbstoffe wie zum Beispiel Acid Brown 4 (C.I. 14

805) und Acid Blue 135 (C.I. 13 385), Anthrachinonfarbstoffe wie zum Beispiel Disperse Violet 4 (C.I. 61 105), Disperse Blue 1 (C.I. 64 500), Disperse Red 15 (C.I. 60 710), Disperse Violet 1 (C.I. 61 100), außerdem 1,4,5,8-Tetraamino-anthrachinon und 1,4-Diamino-anthrachinon enthalten sein.

Weitere geeignete direkt auf das Haar aufziehende Farbstoffe sind beispielsweise in dem Buch von J. C. Johnson, "Hair Dyes", Noyes Data Corp., Park Ridge, USA (1973), Seiten 3 bis 91 und 113 bis 139 (ISBN: 0-8155-0477-2) beschrieben.

Das neue Haarfärbemittel kann weiterhin auch mit sich selbstkuppelnde Farbvorstufen, wie zum Beispiel 2-Amino-5-methyl-phenol, 2-Amino-6-methyl-phenol und 2-Amino-5-ethoxy-phenol, enthalten.

Die Gesamtmenge der direktziehenden Farbstoffe und der selbstkuppelnden Farbvorstufen soll etwa 0,1 bis 3,0 Gewichtsprozent betragen.

Darüberhinaus können in dem Haarfärbemittel noch weitere für solche Mittel übliche Zusätze, beispielsweise Antioxidantien wie Ascorbinsäure oder Natriumsulfit, Parfümöle, Komplexbildner, Netzmittel, Emulgatoren, Verdicker, Pflegestoffe und andere, enthalten sein.

Die Zubereitungsform kann beispielsweise eine Lösung, insbesondere eine wäßrige oder wäßrig-alkoholische Lösung, sein. Die besonders bevorzugten Zubereitungsformen sind jedoch eine Creme, ein Gel oder eine Emulsion.

Seine Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol und Isopropanol, sowie mehrwertige Alkohole wie Ethylenglykol, 1,2-Propylenglykol und Glycerin, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Fettsäuretauride, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie beispielsweise höhere Fettalkohole, Stärke, Polyacrylsäure, Cellulosederivate, Alginate, Vaseline, Paraffinöl und Fettsäuren sowie außerdem Pflegestoffe, wie zum Beispiel Lanolinderivate, Cholesterin, Pantothensäure und Betain.

Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, während die Verdicker in einer Menge von etwa 0,1 bis 25 Gewichtsprozent und die Pflegestoffe in einer Menge von etwa 0,1 bis 5 Gewichtsprozent in den Zubereitungen enthalten sein können.

Je nach Zusammensetzung kann das erfindungsgemäße Haarfärbemittel schwach sauer, neutral oder alkalisch reagieren. Insbesondere weist es einen pH-Wert im alkalischen Bereich zwischen 8,0 und 11,5 auf, wobei die Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid, Verwendung finden.

Bei dem erfindungsgemäßen Verfahren zur oxidativen Färbung von Haaren vermischt man das vorstehend beschriebene Haarfärbemittel unmittelbar vor dem Gebrauch mit einem Oxidationsmittel und trägt eine für die Haarbehandlung ausreichende Menge, je nach Haarfülle, im allgemeinen etwa 50 bis 150 g, dieses Gemisches auf das Haar auf. Als Oxidationsmittel zur Entwicklung der Haarfärbung kommen hauptsächlich Wasserstoffperoxid, beispielsweise als 6prozentige wäßrige Lösung, beziehungsweise dessen Additionsverbindungen an Harnstoff, Melamin oder Natriumborat in Form der 3 bis 12prozentigen wäßrigen Lösung, in Betracht. Wird eine 6prozentige Wasserstoffperoxidlösung als Oxidationsmittel verwendet, so beträgt das Gewichtsverhältnis zwischen Haarfärbemittel und Oxidationsmittel 5 : 1 bis 1 : 2, vorzugsweise jedoch 1 : 1. Größere Mengen an Oxidationsmittel werden vor allem bei höheren Farbstoffkonzentrationen im Haarfärbemittel oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet. Gegebenenfalls wird im Anschluß an diese Spülung mit einem Shampoo gewaschen und eventuell mit einer schwachen physiologisch verträglichen organischen Säure, wie zum Beispiel Zitronensäure oder Weinsäure, nachgespült. Sodann wird das Haar getrocknet.

Die mit dem 3-(2′,2′,2′-Trifluorethyl)amino-phenolderivat der allgemeinen Formel (I) als Kupplersubstanz erzielbaren Haarfärbungen weisen eine gute Licht-, Dauerwell-, Säure- und Reibechtheit auf. Die Farbtöne sind auch bei Einwirkung von Licht-, chemischen Mitteln und Reibung über einen ausreichenden Zeitraum stabil.

Gegenstand der Erfindung ist ferner ein 3-(2′,2′,2′-Trifluorethyl)amino-phenolderivat der allgemeinen Formel (II)

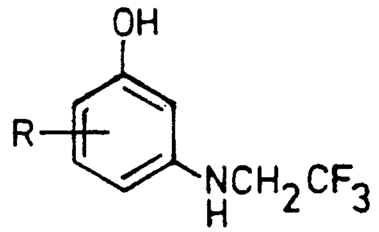

(II),

worin der Rest R

- einen Alkylrest mit 1 bis 4 Kohlenstoffatomen,
- einen Mono- oder Polyhydroxyalkylrest mit 1 bis 4 Kohlenstoffatomen und bis zu 3 Hydroxylgruppen,
- einen Alkoxyrest mit 1 bis 4 Kohlenstoffatomen,
- einen Mono- oder Polyhydroxyalkoxyrest mit 1 bis 4 Kohlenstoffatomen und bis zu 3 Hydroxylgruppen oder
- einen Halogenrest

darstellt, unter der Voraussetzung, daß, falls der Rest R in para-Stellung zur Hydroxylgruppe steht R nicht Alkyl bedeutet.

Beispiele für neue Verbindungen der allgemeinen Formel (I) sind 2-Methyl-3-(2′,2′,2′-trifluorethyl)amino-phenol, 2-Methyl-5-(2′,2′,2′-trifluorethyl)amino-phenol, 2-Chlor-5-(2′,2′,2′-trifluorethyl)amino-phenol, 2-Methoxy-5-(2′,2′,2′-trifluorethyl)amino-phenol und 3-Methyl-5-(2′,2′,2′-trifluorethyl)amino-phenol.

Die Herstellung der bekannten Verbindung 4-Methyl-3-(2′,2′,2′-trifluorethyl)amino-phenol ist in der Literatur E. R. Bissel et al., Journal of Fluorine Chemistry 12, 293 (1978) beschrieben.

Die Verbindungen der Formel (II) wurden hergestellt, indem man ein entsprechend substituiertes Trifluoressigsäureamidderivat mit Natriumborhydrid in Gegenwart einer Lewissäure reduziert.

Die Reduktion wird hierbei in einem cyclischen Ether, beispielsweise Tetrahydrofuran oder Dioxan, als Lösungsmittel durchgeführt.

Als Lewissäure kommen beispielsweise Bortrifluorid-etherat, Aluminiumtrichlorid oder Titantetrachlorid in Betracht.

Die nachfolgenden Beispiele sollen den Gegenstand der Erfindung näher erläutern, ohne ihn darauf zu beschränken.

## Herstellungsbeispiele

**Beispiel 1:** Herstellung von 2-Methyl-5-(2′,2′,2′-trifluorethyl)amino-phenol

Stufe 1: N-(3-Hydroxy-4-methyl)phenyl-2′,2′,2′-trifluor-acetamid

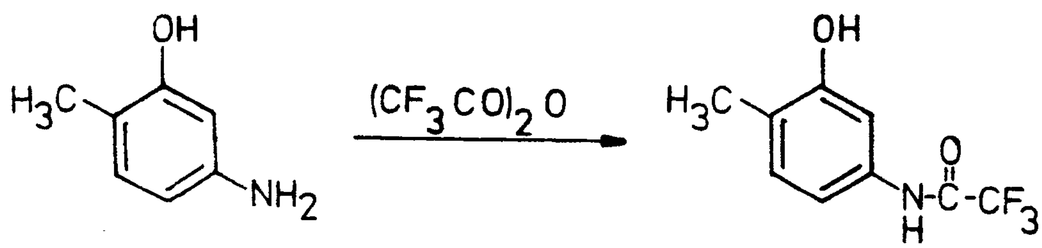


6,16 g (50 mmol) 5-Amino-2-methyl-phenol werden in 100 ml Diethylether gelöst. Dann werden bei Raumtemperatur 10,4 ml (75 mmol; 15,7 g) Trifluoressigsäureanhydrid zugetropft. Sobald die exotherme Reaktion beendet ist, wird der Diethylether im Rotationsverdampfer abdestilliert. Das erhaltene Rohprodukt wird aus einer Ethanol/Wasser-Mischung (1 : 1) umkristallisiert. Man erhält 8,0 g (73 % der Theorie) eines braunen Pulvers vom Schmelzpunkt 130 - 133 Grad Celsius.

| CHN-Analyse: | | % C | % H | % N |
|---|---|---|---|---|
| $C_9H_8F_3NO_2$ | berechnet: | 49,32 | 3,68 | 6,39 |
| | gefunden: | 49,65 | 3,78 | 6,35 |

Stufe 2: 2-Methyl-5-(2',2',2'-trifluorethyl)amino-phenol

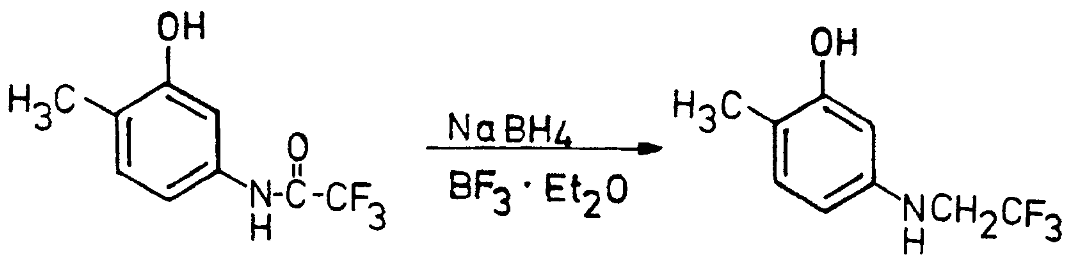


2,2 g (10 mmol) Produkt aus Stufe 1 werden in 20 ml Tetrahydrofuran gelöst und über einem Molekularsieb (3 Angström) getrocknet. Man filtriert, gibt portionswiese unter Stickstoff 0,75 g (20 mmol) Natriumborhydrid zu und läßt eine halbe Stunde bei Raumtemperatur rühren. Nun werden bei Raumtemperatur 3,8 g (3,4 ml; 27 mmol) Bortrifluoridetherat während einer Stunde zugetropft. Danach wird der Ansatz eine weitere Stunde auf 60 Grad Celsius erwärmt. Nach dem Abkühlen auf Raumtemperatur wird die Mischung mit 10 ml einer Tetrahydrofuran/Wasser-Mischung (1 : 1) hydrolysiert. Die Lösung wird mit 7 ml halbkonzentrierter Salzsäure unter Kühlung mit einem Eisbad angesäuert. Nach einer Einwirkungszeit von 30 Minuten wird die Lösung mit 30prozentiger Natronlauge auf den pH-Wert 7 eingestellt und dreimal mit je 30 ml Diethylether extrahiert. Die Etherphase wird mit gesättigter Kochsalzlösung neutral gewaschen und über Magnesiumsulfat getrocknet. Nach Filtration und Abdestillieren der Lösungsmittel wird das zurückbleibende Öl im Kugelrohr destilliert. Man erhält 1,81 g (88 % der Theorie) eines allmählich erstarrenden Öls. Das Produkt hat einen Schmelzpunkt von 69 - 71 Grad Celsius.

| CHN-Analyse | | % C | % H | % N |
|---|---|---|---|---|
| $C_9H_{10}F_3NO$ | berechnet: | 52,69 | 4,91 | 6,83 |
| | gefunden: | 53,76 | 5,10 | 7,08 |

**Beispiel 2 - 5:** Die folgenden Verbindungen wurden analog der in Beispiel 1 gegebenen Vorschrift hergestellt:

**2:** Stufe 1: N-(3-Hydroxy-5-methyl)phenyl-2',2',2'-trifluoracetamid
Schmelzpunkt: 116 - 118 Grad Celsius
Stufe 2: 3-Methyl-5-(2',2',2'-trifluorethyl)amino-phenol
Schmelzpunkt: 129 - 131 Grad Celsius

**3:** Stufe 1: N-(3-Hydroxy-2-methyl)phenyl-2',2',2'-trifluor-acetamid
Schmelzpunkt 114 - 116 Grad Celsius
Stufe 2: 2-Methyl-3-(2',2',2'-trifluorethyl)amino-phenol
Schmelzpunkt: 119 - 121 Grad Celsius

**4:** Stufe 1: N-(3-Hydroxy-4-methoxy)phenyl-2',2',2'-trifluor-acetamid
Schmelzpunkt: 140 - 142 Grad Celsius
Stufe 2: 2-Methoxy-5-(2',2',2'-trifluorethyl)amino-phenol
Schmelzpunkt: 70 - 71 Grad Celsius

**5:** Stufe 1: N-(5-Hydroxy-2-methyl)phenyl-2',2',2'-trifluoracetamid
Schmelzpunkt: 104 - 106 Grad Celsius

Stufe 2: 4-Methyl-3-(2',2',2'-trifluorethyl)amino-phenol
Schmelzpunkt: 64 - 66 Grad Celsius

**Beispiel 6:** Herstellung von 2-Chlor-5-(2',2',2'-trifluorethyl)amino-phenol

Stufe 1: N-(4-Chlor-3-hydroxy)phenyl-2',2',2'-trifluoracetamid

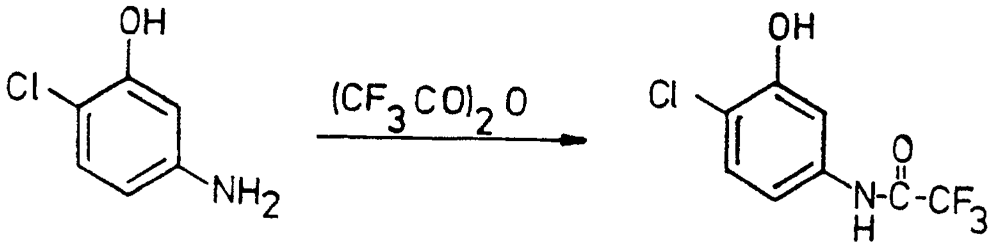


6,0 g (42 mmol) 5-Amino-2-chlor-phenol werden in 75 ml Diethylether gelöst und bei Raumtemperatur tropfenweise mit 10,2 ml (15,4 g; 73 mmol) Trifluoressigsäureanhydrid versetzt. Nachdem die exotherme Reaktion beendet ist, wird der Diethylether abdestilliert. Das erhaltene Rohprodukt (15,5 g) wird aus 20 ml einer Ethanol/Wasser-Mischung (2 : 1) umkristallisiert. Man erhält 3,0 g (91 % der Theorie) weißes Pulver vom Schmelzpunkt 145 - 147 Grad Celsius.

| CHN-Analyse: | | % C | % H | % N |
|---|---|---|---|---|
| $C_8H_5ClF_3NO_2$ | berechnet: | 40,11 | 2,11 | 5,85 |
| | gefunden: | 40,10 | 1,92 | 5,85 |

Stufe 2: 2-Chlor-5-(2',2',2'-trifluorethyl)amino-phenol

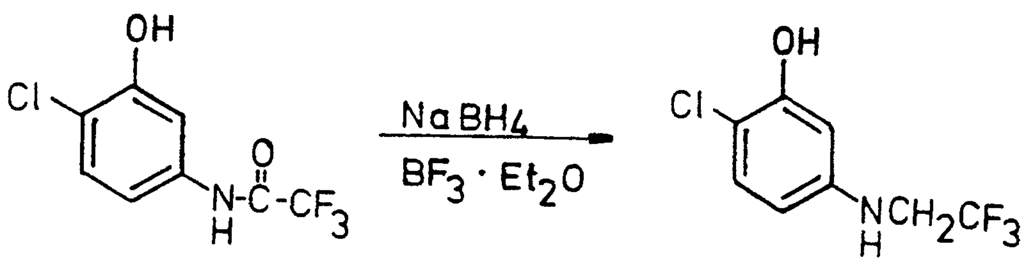


2,0 g (8,3 mmol) Produkt aus Stufe 1 werden in 14 ml Tetrahydrofuran gelöst und über einem Molekularsieb (3 Angström) getrocknet. Man filtriert, gibt portionsweise unter Stickstoff 0,691 g (16,6 mmol) Natriumborhydrid zu und rührt eine halbe Stunde bei Raumtemperatur. Nun werden bei Raumtemperatur 3,53 g (2,34 ml; 25 mmol) Bortrifluoridetherat während einer Stunde zugetropft. Danach wird der Ansatz eine weitere Stunde auf 60 Grad Celsius erwärmt. Nach dem Abkühlen wird die Mischung mit 6,8 ml einer Tetrahydrofuran/Wasser-Mischung (1 : 1) hydrolysiert. Die Lösung wird mit 4 ml halbkonzentrierter Salzsäure unter Kühlung mit einem Eisbad angesäuert. Nach einer Einwirkungszeit von 30 Minuten wird die Lösung mit 30prozentiger Natronlauge auf den pH-Wert 7 eingestellt und dreimal mit je 30 ml Diethylether extrahiert. Die Etherphase wird mit gesättigter Kochsalzlösung neutral gewaschen und über Magnesiumsulfat getrocknet. Nach Filtration und Abdestillieren der Lösungsmittel wird das zurückbleibende Öl im Kugelrohr destilliert (Siedepunkt 130 Grad Celsius/4 Pa). Man erhält 1,34 g (72 % der Theorie) eines erstarrenden Öls. Das Produkt hat einen Schmelzpunkt von 66 - 67,5 Grad Celsius.

| CHN-Analyse: | | % C | % H | % N |
|---|---|---|---|---|
| $C_8H_7ClF_3NO$ | berechnet: | 42,59 | 3,13 | 6,21 |
| | gefunden: | 42,71 | 3,36 | 6,22 |

**BEISPIELE FÜR HAARFÄRBEMITTEL:**

**Beispiel 7:** Haarfärbemittel in Cremeform

| | |
|---|---|
| 1,0 g | 2-Methyl-5-(2',2',2'-trifluorethyl)amino-phenol |
| 1,1 g | 2-(2',5'-Diaminophenyl)ethanol-sulfat |
| 0,3 g | Natriumsulfit, wasserfrei |
| 3,5 g | Laurylalkohol-diglykolethersulfat, Natrium-salz (28prozentige wäßrige Lösung) |
| 15,0 g | Cetylalkohol |
| 4,0 g | Ammoniak, 25prozentige wäßrige Lösung |
| 75,1 g | Wasser vollentsalzt |
| 100,0 g | |

50 g des vorstehenden Haarfärbemittels werden unmittelbar vor der Anwendung mit 50 g Wasserstoffperoxidlösung (6prozentig) vermischt und das Gemisch auf gebleichtes Haar aufgetragen. Nach einer Einwirkungszeit von 30 Minuten bei 40 Grad Celsius wird das Haar ausgespült, mit einem Shampoo gewaschen und getrocknet. Das Haar ist rotviolett gefärbt.

**Beispiel 8:** Haarfärbelösung

| | |
|---|---|
| 0,63 g | 2-Chlor-5-(2',2',2'-trifluorethyl)amino-phenol |
| 0,61 g | 2,5-Diaminotoluol-sulfat |
| 10,00 g | Laurylalkohol-diglykolethersulfat, Natrium-salz (28prozentige wäßrige Lösung) |
| 10,00 g | Isopropanol |
| 0,30 g | Ascorbinsäure |
| 10,00 g | Ammoniak, 25prozentige wäßrige Lösung |
| 68,46 g | Wasser vollentsalzt |
| 100,0 g | |

50 g des vorstehenden Haarfärbemittels werden unmittelbar vor der Anwendung mit 50 g Wasserstoffperoxidlösung (6prozentig) vermischt und das Gemisch auf gebleichtes Haar aufgetragen. Nach einer Einwirkungszeit von 30 Minuten bei 40 Grad Celsius wird das Haar ausgespült, mit einem Shampoo gewaschen und getrocknet. Das Haar ist tief blauschwarz gefärbt.

**Beispiel 9:** Haarfärbegel

| | |
|---|---|
| 2,5 g | 2-Chlor-5-(2',2',2'-trifluorethyl)amino-phenol |
| 1,2 g | 4-Aminophenol |
| 0,3 g | Ascorbinsäure |
| 15,0 g | Ölsäure |
| 7,0 g | Isopropanol |
| 9,0 g | Ammoniak 25prozentige wäßrige Lösung |
| 65,0 g | Wasser vollentsalzt |
| 100,0 g | |

50 g des vorstehenden Haarfärbemittels werden unmittelbar vor der Anwendung mit 50 g Wasserstoffperoxidlösung (6prozentig) vermischt und das Gemisch auf gebleichtes Haar aufgetragen. Nach einer Einwirkungszeit von 30 Minuten bei 40 Grad Celsius wird das Haar ausgespült, mit einem Shampoo gewaschen und getrocknet. Das Haar hat einen roten Farbton angenommmen.

**Beispiel 10:** Haarfärbemittel in Cremeform

| | |
|---|---|
| 0,9 g | 2-Chlor-5-(2',2',2'-trifluorethyl)amino-phenol |
| 2,0 g | 2,5-Diaminotoluol-sulfat |
| 0,7 g | 5-(2-Hydroxyethyl)amino-benzodioxol-(1,3) |
| 0,3 g | Natriumsulfit, wasserfrei |
| 3,5 g | Laurylalkohol-diglykolethersulfat, Natriumsalz (28prozentige wäßrige Lösung) |
| 15,0 g | Cetylalkohol |
| 4,0 g | Ammoniak, 25prozentige wäßrige Lösung |
| 73,6 g | Wasser vollentsalzt |
| 100,0 g | |

50 g des vorstehenden Haarfärbemittels werden unmittelbar vor der Anwendung mit 50 g Wasserstoffperoxidlösung (6prozentig) vermischt und das Gemisch auf gebleichtes Haar aufgetragen. Nach einer Einwirkungszeit von 30 Minuten bei 40 Grad Celsius wird das Haar ausgespült, mit einem Shampoo gewaschen und getrocknet. Das Haar ist schwarz gefärbt.

**Beispiel 11 - 14:** Haarfärbelösung

Es werden Haarfärbelösungen gemäß Beispiel 8 hergestellt, worin die Kupplersubstanz 2-Chlor-5-(2',2',2'-trifluorethyl)amino-phenol durch jeweils eine der folgenden erfindungsgemäßen Kupplersubstanzen ersetzt ist. Mit den so erhaltenen Haarfärbelösungen wird eine Haarbehandlung, wie sie in Beispiel 8 beschrieben ist, durchgeführt:

**11:** 3-Methyl-5-(2',2',2'-trifluorethyl)amino-phenol Nach der Haarbehandlung sind die Haare rotbraun gefärbt.

**12:** 4-Methyl-3-(2',2',2'-trifluorethyl)amino-phenol Nach der Haarbehandlung sind die Haare rotgrau gefärbt.

**13:** 2-Methyl-3-(2',2',2'-trifluorethyl)amino-phenol Nach der Haarbehandlung sind die Haare kardinalrot gefärbt.

**14:** 2-Methoxy-5-(2 ,2',2'-trifluorethyl)amino-phenol Nach der Haarbehandlung sind die Haare rotviolett gefärbt.

**Beispiel 15:** Haarfärbelösung

| | |
|---|---|
| 0,63 g | 2-Chlor-5-(2',2',2'-trifluorethyl)amino-phenol |
| 0,64 g | Tetraaminopyrimidin-sulfat-monohydrat |
| 10,00 g | Laurylalkohol-diglykolethersulfat, Natrium-salz (28prozentige wäßrige Lösung) |
| 10,00 g | Isopropanol |
| 0,30 g | Ascorbinsäure |
| 10,00 g | Ammoniak, 25prozentige wäßrige Lösung |
| 68,43 g | Wasser vollentsalzt |
| 100,0 g | |

50 g des vorstehenden Haarfärbemittels werden unmittelbar vor der Anwendung mit 50 g Wasserstoffperoxidlösung (6prozentig) vermischt und das Gemisch auf gebleichtes Haar aufgetragen. Nach einer Einwirkungszeit von 30 Minuten bei 40 Grad Celsius wird das Haar ausgespült, mit einem Shampoo gewaschen und getrocknet. Das Haar ist stahlblau gefärbt.

Alle Prozentangaben dieser Anmeldung stellen, soweit nicht anders angegeben, Gewichtsprozente dar.

## Ansprüche

1. Mittel zur oxidativen Färbung von Haaren auf der Basis einer Entwicklersubstanz-Kupplersubstanz-Kombination, dadurch gekennzeichnet, daß es als Kupplersubstanz ein 3-(2',2',2'-Trifluorethyl)amino-phenolderivat der allgemeinen Formel (I)

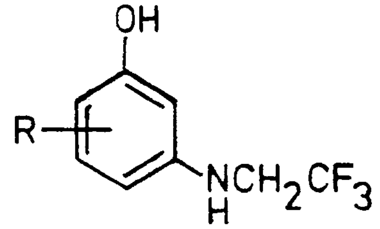

(I),

worin der Rest R
- einen Alkylrest mit 1 bis 4 Kohlenstoffatomen,
- einen Mono- oder Polyhydroxyalkylrest mit 1 bis 4 Kohlenstoffatomen und bis zu 3 Hydroxylgruppen,
- einen Alkoxyrest mit 1 bis 4 Kohlenstoffatomen,
- einen Mono- oder Polyhydroxyalkoxyrest mit 1 bis 4 Kohlenstoffatomen und bis zu 3 Hydroxylgruppen oder
- einen Halogenrest

darstellt, oder dessen physiologisch verträgliches Salz enthält.

**2.** Mittel nach Anspruch 1, dadurch gekennzeichnet, daß der Rest R in para-Stellung zum (2',2',2'-Trifluorethyl)aminorest steht.

**3.** Mittel nach den Ansprüchen 1 und 2, dadurch gekennzeichnet, daß die Kupplersubstanz der Formel (I) in einer Menge von 0,01 bis 4,0 Gewichtsprozent, vorzugsweise 0,02 bis 2,0 Gewichtsprozent, enthalten ist.

**4.** Mittel nach den Ansprüchen 1 bis 3, dadurch gekennzeichnet, daß mindestens eine der Entwicklersubstanzen 1,4-Diamino-benzol, 2,5-Diamino-toluol, 2,5-Diamino-anisol, 4-Amino-phenol, 2,5-Diamino-benzylalkohol, 2-(2',5'-Diamino)phenyl-ethanol, 3-Methyl-4-amino-phenol oder Tetraaminopyrimidin enthalten ist.

**5.** Mittel nach den Ansprüchen 1 bis 4, dadurch gekennzeichnet, daß zusätzlich mindestens eine der Kupplersubstanzen Resorcin, 4-Chlor-resorcin, 4,6-Dichlor-resorcin, 2-Methyl-resorcin, 2-Amino-4-(2'-hydroxyethyl)amino-anisol, 1,5-Dihydroxy-tetralin, 3-Amino-2-methyl-phenol, 2,4-Diamino-anisol, 2,4-Diamino-benzylalkohol, 5-Amino-2-methyl-phenol, 2,4-Diamino-phenetol, 1,3-Diamino-benzol, 1-Naphthol, 4-Hydroxy-in dol, 5-Hydroxy-benzodioxol-(1,3), 5-Amino-benzodioxol-(1,3), 5-(2'-Hydroxyethyl)-amino-benzodioxol-(1,3), 2,4-Dihydroxy-anisol und 2,4-Dihydroxy-phenoxyethanol enthalten ist.

**6.** Mittel nach den Ansprüchen 1 bis 5, dadurch gekennzeichnet, daß die Gesamtmenge der darin enthaltenen Entwicklersubstanz-Kupplersubstanz-Kombination 0,1 bis 6,0 Gewichtsprozent, vorzugsweise 0,5 bis 3,0 Gewichtsprozent, beträgt.

**7.** Mittel nach den Ansprüchen 1 bis 6, dadurch gekennzeichnet, daß es mindestens einen der direkt auf das Haar aufziehenden Farbstoffe Diamond Fuchsine (C.I. 42 510), Leather Ruby HF (C.I. 42 520), 2-Amino-4,6-dinitro-phenol, 2-(2'-Hydroxyethyl)amino-4,6-dinitro-phenol, 4-(2'-Hydroxyethyl)amino -2-nitro-anilin, 2 Amino-4-nitro-phenol, Acid Brown 4 (C.I. 14 805), Acid Blue 135 (C.I. 13 385), Disperse Violet 4 (C.I. 61 105), Disperse Blue 1 (C.I. 64 500), Disperse Red 15 (C.I. 60 710), Disperse Violet 1 (C.I. 61 100), 1,4,5,8-Tetraamino-antrachinon oder 1,4-Diamino-anthrachinon enthält.

**8.** Mittel nach den Ansprüchen 1 bis 7, dadurch gekennzeichnet, daß es zusätzlich 2-Amino-5-methyl-phenol, 2-Amino-6-methyl-phenol oder 2-Amino-5-ethoxy-phenol enthält.

**9.** Verfahren zum oxidativen Färben von Haaren, dadurch gekennzeichnet, daß man ein Mittel nach den Ansprüchen 1 bis 8 unmittelbar vor dem Gebrauch mit einem Oxidationsmittel, vorzugsweise Wasserstoffperoxid als 6prozentige wäßrige Lösung, vermischt, anschließend eine für die Haarbehandlung ausreichende Menge dieses Gemisches auf das Haar aufträgt und bei 15 bis 50 Grad Celsius 10 bis 45 Minuten lang auf das Haar einwirken läßt, sodann das Haar mit Wasser spült und trocknet.

**10.** 3-(2',2',2'-Trifluorethyl)amino-phenolderivat der allgemeinen Formel (II)

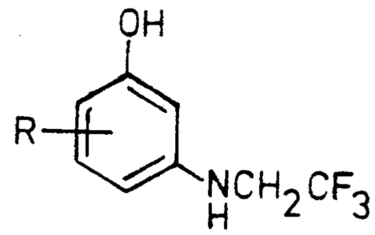

(II),

worin der Rest R

- einen Alkylrest mit 1 bis 4 Kohlenstoffatomen,
- einen Mono- oder Polyhydroxyalkylrest mit 1 bis 4 Kohlenstoffatomen und bis zu 3 Hydroxylgruppen,
- einen Alkoxyrest mit 1 bis 4 Kohlenstoffatomen,
- einen Mono- oder Polyhydroxyalkoxyrest mit 1 bis 4 Kohlenstoffatomen und bis zu 3 Hydroxylgruppen oder
- einen Halogenrest

darstellt, unter der Voraussetzung, daß, falls der Rest R in para-Stellung zur Hydroxylgruppe steht R nicht Alkyl bedeutet.

**11.** 2-Methyl-3-(2 ,2′,2′-trifluorethyl)amino-phenol

**12.** 2-Methyl-5-(2′,2′,2′-trifluorethyl)amino-phenol

**13.** 2-Chlor-5-(2′,2′,2′-trifluorethyl)amino-phenol

**14.** 2-Methoxy-5-(2′,2′,2′-trifluorethyl)amino-phenol

**15.** 3-Methyl-5-(2′,2′,2′-trifluorethyl)amino-phenol

## Claims

**1.** An agent for the oxidative colouring of hair, based on a developer substance-coupler substance combination, characterised in that it contains as a coupler substance a 3-(2',2',2'-trifluoroethyl)amino-phenol-derivative to the general formula (I)

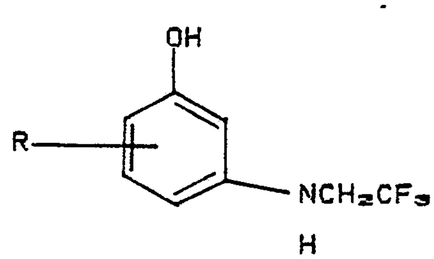

(I),

in which the radical R represents

- an alkyl radical containing 1 to 4 carbon atoms,
- a mono or polyhydroxyalkyl radical containing 1 to 4 carbon atoms and up to 3 hydroxyl groups,
- an alkoxy radical containing 1 to 4 carbon atoms,
- a mono or polyhydroxyalkoxy radical containing 1 to 4 carbon atoms and up to 3 hydroxyl groups or
- a halogen radical,

or a physiologically-acceptable salt thereof.

**2.** An agent according to Claim 1, characterised in that the radical R is in the para position relative to the

(2',2',2'-trifluroethyl)amino radical.

**3.** An agent according to Claims 1 and 2, characterised in that the coupler substance in Formula (I) is contained in a quantity of 0.01 to 4.0% by weight and preferably 0.02 to 2.0% by weight.

**4.** An agent according to Claims 1 to 3, characterised in that at least one of the developer substances is 1,4-diamino-benzene, 2,5-diamino-toluene, 2,5-diamino-anisole, 4-amino-phenol, 2,5-diamino-benzyl alcohol, 2-(2',5'-diamino)phenylethanol, 3-methyl-4-amino-phenol or tetraaminopyrimidine.

**5.** An agent according to Claims 1 to 4, characterised in that it contains additionally at least one of the coupler substances resorcinol, 4-chloro-resorcinol, 4,6-dichloro-resorcinol, 2-methyl-resorcinol, 2-amino-4-(2'-hydroxyethyl)-amino-anisole, 1,5-dihydroxy-tetralin, 3-amino-2-methyl-phenol, 2,4-diamino-anisole, 2,4-diamino-benzyl, alcohol, 5-amino-2-methyl-phenol, 2,4-diamino-phenetol, 1,3-diamino-benzene, 1-naphthol, 4-hydroxy-indole, 5-hydroxy-benzodioxol-(1,3), 5-amino-benzodioxol-(1,3), 5-(2'-hydroxyethyl)-amino-benzodioxol-(1,3), 2,4-dihydroxy-anisole and 2,4-dihydroxy-phenoxy ethanol.

**6.** An agent according to Claims 1 to 5, characterised in that the total quantity of the developer substance-coupler substance combination which is then contained therein amounts to 0.1 to 6.0 per cent by weight and preferably 0.5 to 3.0 per cent by weight.

**7.** An agent according to Claims 1 to 6, characterised in that it contains at least one of the following dyes which are applied directly to the hair: Diamond Fuchsin (C.I. 42 510), Leather Ruby HF (C.I. 42 520), 2-amino-4,6-dinitro-phenol, 2-(2'-hydroxyethyl)amino-4,6-dinitro-phenol, 4-(2'-hydroxyethyl)amino -2-nitro-aniline, 2 amino-4-nitro-phenol, Acid Brown 4 (C.I. 14 805), Acid Blue 135 (C.I. 13 385), Disperse Violet 4 (C.I. 61 105), Disperse Blue 1 (C.I. 64 500), Disperse Red 15 (C.I. 60 710), Disperse Violet 1 (C.I. 61 100), 1,4,5,8-tetraamino-anthraquinone or 1,4-diamino-anthraquinone.

**8.** An agent according to Claims 1 to 7, characterised in that it contains additionally 2-amino-5-methyl-phenol 2-amino-6-methyl-phenol or 2-amino-5-ethoxy-phenol.

**9.** A method for the oxidative colouring of hair, characterised in that an agent according to Claims 1 to 8 is, immediately prior to use, mixed with an oxidising agent, preferably hydrogen peroxide in the form of a 6 percent aqueous solution, after which a quantity of this mixture which is adequate for treating the hair is applied to the hair and allowed to act on the hair at 15 to 50 degrees C for 10 to 45 minutes after which the hair is rinsed with water and dried.

**10.** 3-(2',2',2'-trifluoroethyl)amino-phenol derivative to the general formula (II)

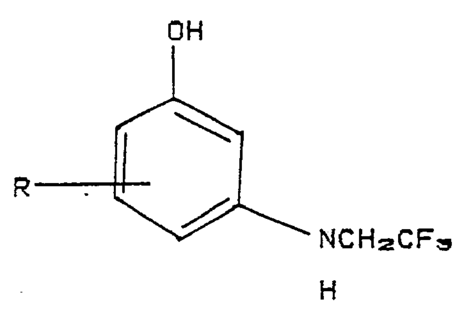

(II),

in which the radical R denotes

- an alkyl radical containing 1 to 4 carbon atoms,
- a mono or polyhydroxy alkyl radical with 1 to 4 carbon atoms and up to 3 hydroxyl groups,
- an alkoxy radical with 1 to 4 carbon atoms,
- a mono- or polyhydroxy alkoxy radical with 1 to 4 carbon atoms and up to 3 hydroxyl groups or
- a halogen radical,

subject to R not denoting alkyl if the radical R is in the para position relative to the hydroxyl group.

**11.** 2-methyl-3-(2,2',2'-trifluoroethyl)amino-phenol.

**12.** 2-methyl-5-(2',2',2'-trifluoroethyl)amino-phenol.

**13.** 2-chloro-5-(2',2',2'-trifluoroethyl)amino-phenol.

**14.** 2-methoxy-5-(2',2',2'-trifluoroethyl)amino-phenol.

**15.** 3-methyl-5-(2',2',2'-trifluoroethyl)amino-phenol.

## Revendications

**1.** Agent de coloration oxydative des cheveux à base d'une combinaison substance révélateur-substance de couplage, caractérisé en ce que la substance de couplage est de la forme d'un dérivé 3-(2',2',2'-trifluoréthyl)amino-phénol de formule générale (1) :

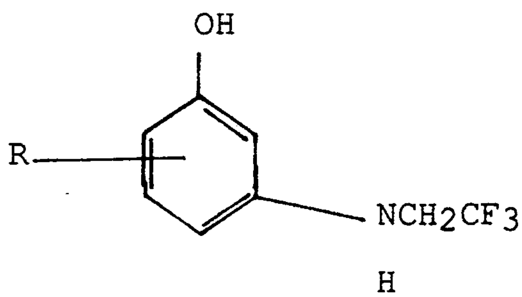

(1)

dans laquelle le radical R représente
- un radical alcoyle possèdant de 1 à 4 atomes de carbone.
- un radical mono- ou polyhydroxyle-alcoyle, possèdant de 1 à 4 atomes de carbone et jusqu'à 3 groupes hydroxyles.
- un radical alkoxyle possèdant de 1 à 4 atomes de carbone.
- un radical mono- ou polyhydroxyle-alkoxyle, possèdant de 1 à 4 atomes de carbone et jusqu'à 3 groupes hydroxyles.

ou bien
- un radical halogène,

ou bien ce radical R contient leur sel, physiologiquement compatible.

**2.** Agent selon la revendication 1, caractérisé en ce que le raical R est en position para, vis-à-vis du radical (2',2',2'-trifluoréthylamine.

**3.** Agent selon les revendications 1 et 2, caractérisé en ce que la substance de couplage de formule (1) est contenue à proportion de 0,01 à 4,0 pourcent en poids, de préférence de 0,02 à 2,0 pourcent en poids.

**4.** Agent selon les revendications 1 à 3, caractérisé en ce qu'il est contenu au moins l'une des substances révélateur suivantes : 1,4-diamino-benzol, 2,5 diamino-toluol, 2,5-diamino-anysol, 4-amino-phénol, 2,5-diamino-benzylalcool, 2-(2',5'-diamino)pnéhyl-éthanol, 3-méthyl-4-amino-phénol ou bien tétraaminopyromidin.

**5.** Agent selon les revendications 1 à 4, caractérisé en ce qu'il est contenu additionnel au moins l'une des substances de couplage suivantes : Résorcine, 4-chlororésorcine, 4,6-dichlororésorcine,
2-méthylrésorcine, 2-amino-4-(2'-hydroxyléthyl)amino-anysol,
1,5-dihydroxytétraline, 3-amino-2-méthyl-phénol,
2,4-diamino-anisol, 2,4-diamino-alcool benzilique,
5-amino-2-méthyl-phénol, 2,4-diamino-phénétol,

1,3-diamino-benzol, 1-naphtol, 4-hydroxy-in dol, 5-hydroxy-benzodioxol-(1,3), 5-amino-benzodioxol-(1,3), 5-(2-hydroxyéthylamino-benzodioxol-(1,3), 2,4-dihydroxy-anisol et 2,4-dihydroxy-phénoxyéthanol.

**6.** Agent selon les revendications 1 à 5, caractérisé en ce que la quantité totale de la combinaison substance révélateursubstance de couplage qu'il contient s'élève de 0,1 à 6,0 en pourcentage poids, de préférence de 0,5 à 3,0 en pourcentage poids.

**7.** Agent selon les revendications 1 à 6, caractérisé en ce qu'il contient au moins l'un des colorants suivants colorant les cheveux :
Diamond fuchsine (C.I. 42 510), Leather Ruby HF (C.I. 42 520),
2-amino-4,6-dinitro-phénol,
2-(2'-hydroxyéthyl)amino-4,6-donitro-phénol,
4-(2'-hydroxyéthyl)amino-2-nitro-aniline,
2-amino-4-nitro-phénol, acid brown (C.I. 14 805),
acid blue 135 (C.I.13 385), disperse violet 4 (C.I.61 105), disperse blue 1 (C.I. 64 500), disperse red 15 (C.I. 60 710), disperse violet 1 (C.I. 61 100), 1,4,5,8-tétraamino-antraquinone ou bien 1,4-diamino-antraquinone.

**8.** Agent selon les revendications 1 à 7, caractérisé en ce qu'il contient additionnel 2-amino-5-méthyl-phénol, 2-amino-6-méthyl-phénol, ou bien 2-amino-5-éthoxy-phénol.

**9.** Agent de coloration oxydative des cheveux, caractérisé en ce que l'on mélange un agent, selon revendications 1 à 8, directement avant l'utilisation, avec un agent d'oxydation,de préférence du peroxyde d'hydrogène, autrement dénommé eau oxygénée, sous forme de solution aqueuse à 6 pourcent, que l'on applique immédiatement après une quantité suffisante de ce mélange sur les cheveux, pour effectuer le traitement des cheveux, et que l'on laisse agir sur les cheveux à une température comprise entre 15 et 50 °C, pendant 10 à 45 minutes, les cheveux étant ensuite rinçés à l'eau et séchés.

**10.** Le dérivé 3-(2',2',2'-trifluoréthyl)amino-phénol est de formule générale (II) :

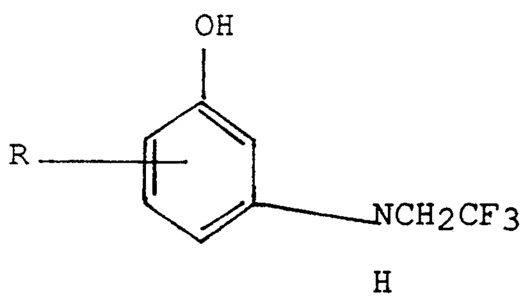

(II)

où le radical R représente :

- un radical alcoyle possèdant de 1 à 4 atomes de carbone.
- un radical mono- ou polyhydroxyle-alcoyle, possèdant de 1 à 4 atomes de carbone et jusqu'à 3 groupes hydroxyles.
- un radical alkoxyle possèdant de 1 à 4 atomes de carbone.
- un radical mono- ou polyhydroxyle-alkoxyle, possèdant de 1 à 4 atomes de carbone et jusqu'à 3 groupes hydroxyles.

ou bien

- un radical halogène,

tout ceci sous la condition que la radical R ne signifie pas alcoyle, au cas où le radical R est en position para, par rapport au groupe hydroxyle,

**11.** 2-méthyl-3-(2',2',2'-trifluoréthyl)amino-phénol.

**12.** 2-méthyl-5-(2',2',2'-trifluoréthyl)amino-phénol.

**13.** 2-chloro-5-(2',2',2'-trifluoréthyl)amino-phénol.

**14.** 2-methoxy-5-(2',2',2'-trifluoréthyl)amino-phénol.

**15.** 3-méthyl-5-(2',2',2'-trifluoréthyl)amino-phénol.